# EUROPEAN PATENT APPLICATION

(11) **EP 3 072 481 A2**
(43) Date of publication of application: **28.09.2016**
(21) Application number: 16160990.4
(22) Date of filing: 17.03.2016
(51) Int. Cl.: A61F 5/058

(54) **AN APPARATUS AND METHOD FOR SUPPORTING A LEG OF A PERSON LAID ON A SURFACE**

(30) Priority: 23.03.2015 GB 201504827
(71) Applicant: Talar-Made Limited, Derbyshire S41 9RN (GB)
(72) Inventor: CREWDSON, Bernard Martin, Chesterfield, Derbyshire S41 9RN (GB)
(74) Representative: Swindell & Pearson Limited

(57) **Abstract**

An apparatus (101) for supporting a leg of a person laid on a surface. The apparatus comprises a main body (102) for location around a leg of a person and a fastening mechanism (104) configured to connect different parts of the main body (102) for attaching the apparatus to a leg. The fastening mechanism (104) comprises an engaging element (105) and a hole (106) configured to receive the engaging element.

## Description

### FIELD OF THE INVENTION

Embodiments of the present invention relate to an apparatus and method for supporting a leg of a person laid on a surface. In particular, they relate to an apparatus and method for supporting a leg of a person laid on a surface such as a bed to ensure the person's heel is held above the surface.

### BACKGROUND TO THE INVENTION

Apparatuses, known as heel protectors, or suspension boots, are used for supporting a leg of a person laid on a surface. Such apparatuses may be used when, for medical reasons, it is desirable to hold the heel of the person above a surface, such as the upper surface of a bed, to avoid pressure being applied to the heel. Known apparatuses comprise a deformable main body that provides a cushioned support to the lower leg of the person. A problem with such apparatuses is that they are provided with a hook and loop fastening mechanism which may become dirty, harbor bacteria and which cannot easily be cleaned.

### BRIEF DESCRIPTION OF VARIOUS EMBODIMENTS OF THE INVENTION

According to various, but not necessarily all, embodiments of the invention there is provided an apparatus for supporting a leg of a person laid on a surface, the apparatus comprising: a main body for location around a leg of a person; and a fastening mechanism configured to connect different parts of the main body for attaching the apparatus to a leg, wherein the fastening mechanism comprises an engaging element and a hole configured to receive the engaging element.

This provides the advantage that the fastening mechanism may be easily cleaned.

In some embodiments, the fastening mechanism comprises connection portion formed of a polymeric material attached to the main body.

In some embodiments, the engaging element is a button and the hole is a button hole.

The button may be formed of a polymeric material. The button may be supported on a stalk from a base and the button, the stalk and the base are formed in one piece. The fastening mechanism may comprise a connection portion formed of a polymeric material attached to the main body, and the button hole is formed in the connection portion. A reinforcing element may be attached to the connection portion at the location of the button hole.

The button may be one of a plurality of buttons and the button hole may be one of a plurality of button holes, each button hole configured to receive a button. The number of buttons may be less than the number of button holes, and a button is attachable to a plurality of different button holes

In some embodiments, the main body comprises a left portion and a right portion that are configurable to extend on either side of a space for receiving a foot, and the main body further comprises a sole portion configurable to extend between the left portion and the right portion for supporting a sole of a foot.

The main body may comprise an inflatable item and the sole portion may be inflatable. The sole portion may be releasably attachable to the left portion and/or the right portion by a second fastening mechanism. The second fastening mechanism may comprise buttons and button holes. The main body may be reconfigurable between a first configuration in which the second fastening mechanism is undone and the main body is able to lay flat and a second configuration for use.

In some embodiments, the main body comprises an inflatable item.

The main body may comprise a case containing the inflatable item. The inflatable item may comprise an outer wall defining a cavity for receiving a gas, and the cavity may comprise several compartments such that when a foot is located within the apparatus different compartments are arranged next to different parts of the foot.

The main body may comprise an inflatable item and the inflatable item may comprise an outer wall defining a cavity for receiving a gas, the cavity comprising several compartments and wherein the button may be located on one of the compartments.

According to various, but not necessarily all, embodiments of the invention there is provided a method of supporting a leg of a person laid on a surface, comprising: locating a main body of an apparatus around a leg of a person; and attaching the apparatus to the leg by connecting different parts of the main body by fastening a fastening mechanism, wherein fastening the fastening mechanism comprises locating an engaging element within a hole configured to receive the engaging element.

In an embodiment fastening the fastening mechanism comprises fastening a button in a button hole.

According to various, but not necessarily all, embodiments of the invention there is provided a method of manufacturing an apparatus for supporting a leg of a person laid on a surface, the apparatus comprising: a main body for location around a leg of a person; and a fastening mechanism configured to connect different parts of the main body for attaching the apparatus to a leg, wherein the fastening mechanism comprises an engaging element and a hole configured to receive the engaging element, and the engaging element is formed of a first polymer material and the main body has an outer surface comprising a second polymer material, and wherein the method comprises welding the engaging element to the outer surface.

In some embodiments the engaging element comprises a button supported on a stalk from a base and the welding comprises welding the base to the outer surface.

In some embodiments the method comprises welding a reinforcing element to a connection portion of the apparatus at the location of the button hole.

### BRIEF DESCRIPTION OF THE DRAWINGS

For a better understanding of various examples of embodiments of the present invention reference will now be made by way of example only to the accompanying drawings in which:
Fig. 1 shows a schematic cross-sectional view of an apparatus 101;
Fig. 2 shows an example of apparatus 101 in which the main body 102 comprises an inflatable item 201;
Figs. 3A and 3B show more detailed views of the button 105 (separate from the button hole 106) in cross-section and perspective view respectively;
Figs. 4A and 4B show more detailed views of the button hole 106 (separate from the button 105) in cross-sectional view and perspective view respectively;
Fig. 5 shows a plan view of the apparatus 101 in its flat configuration;
Fig. 6 shows a perspective view of the apparatus 101 in a second configuration;
Fig. 7 shows the apparatus 101 comprising inflatable item 201 in cross-section;
Fig. 8 shows an end portion of compartment 203E of inflatable item 201, the inlet valve 505 and the pressure release valve 506;
Figs. 9A and 9B show an example of a pressure release valve 506 in a front view and cross-sectional view;
Figs. 10A and 10B show an alternative example of an apparatus 101A for supporting a leg of a person laid on a surface 1002 in a perspective view and a cross-sectional view;
Fig. 11 shows the case 1004 of apparatus 101A in an unfastened, flat configuration.

### DETAILED DESCRIPTION OF VARIOUS EMBODIMENTS OF THE INVENTION

The Figures illustrate an apparatus 101 for supporting a lower leg of a person laid on a surface, the apparatus 101 comprising: a main body 102 for location around a leg 103 of a person; and a fastening mechanism 104 configured to connect different parts of the main body for attaching the apparatus to a leg, wherein the fastening mechanism 104 comprises an engaging element 105 and a hole 106 configured to receive the engaging element.

In examples, the engaging element 105 is a button and the hole 106 is a button hole. In alternative examples, the engaging element may be a male part of a different type of fastener and the hole may be defined by a corresponding female part. For example, in an alternative example fastening mechanism may resemble fasteners used on busks, in which a male part comprises a post with an enlarged head and the female part comprises a plate with a keyhole-shaped hole, such that the enlarged head is configured to pass through a wider part of the hole but not through the narrower part. In a further alternative example, the fastening mechanism may comprise a male part in the form of a hook-shaped member which is configured to engage the female part by location of the hook through the hole formed in the female part. It may be noted that in each case the fastening mechanism has a form that has a structure that is relatively easy to clean and does not include inaccessible surfaces such as those found in hook and loop fasteners.

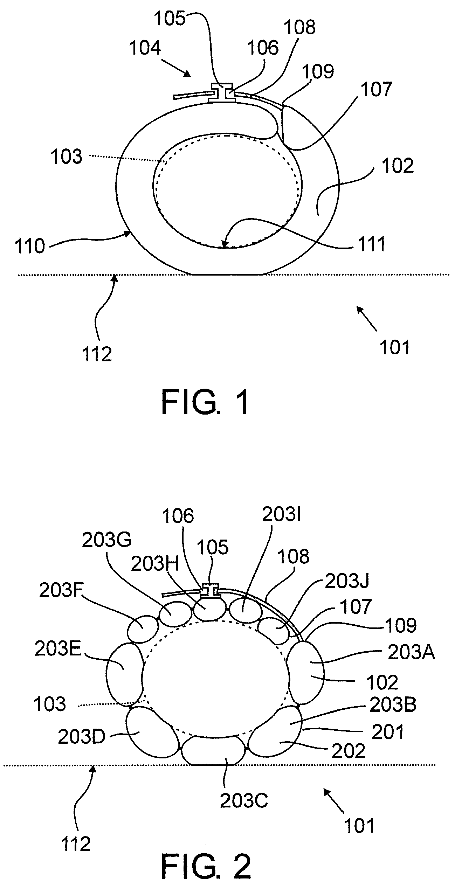

A schematic cross-sectional view of an example of an apparatus 101 is shown in Fig. 1. The main body 102 is configured to be easily folded or bent such that it can be deformed to provide a good fit around a person's lower leg 103. A button 105 is provided on the main body 102 close to one edge 107 of the main body. A button hole 106 is formed in a connecting portion 108 provided at a different edge 109 of the main body. The button 105 is shown fastened in the button hole 106 in Fig. 1, but it should be understood that the button is releasable from the button hole by unfastening.

The main body 102 has an outer surface 110 and an inner surface 111. The outer surface 110, the inner surface 111, the connecting portion 108 and the button 105 are all formed of water impermeable materials so that they may be cleaned, for example by washing and/or wiping. In examples, the outer surface and the inner surface are formed of a material that is microbial resistant. In an example the outer surface and the inner surface are provided by one or more sheets of polyether based thermoplastic polyurethane (TPU).

In embodiments, the button is formed of a polymeric material, such as poly vinyl chloride.

The main body 102 is compressible under pressure, for example as applied by the weight of a person's lower leg. Consequently, the inner surface 111 of the main body 102 is able to be deformed around the leg 103 (shown in dotted outline) under the weight of the leg to provide a relatively large area of contact and relatively low contact pressure between the inner surface and the leg. However, the main body 102 is sufficiently resistant to compression to hold the leg 103 spaced apart from a surface 112 that supports the apparatus 101.

The main body 102 may be formed of various compressible materials, such as natural or synthetic rubber or a polymeric material having a foam structure, but in some examples, the main body comprises an inflatable item.

An example of apparatus 101 is shown in Fig. 2 in which the main body 102 comprises an inflatable item 201. The inflatable item 201 has an outer wall formed of one or more sheets of material, with portions of the material being attached to other portions to form one or more cavities 202 for receiving and containing a gas.

As used herein the word "gas" includes pure gasses such as nitrogen or mixtures of gasses such as air.

Each of the cavities of the inflatable item may be divided into several compartments, such that gas within one of the compartments may flow into other ones of the compartments. For example, ten compartments 203A to 203I are shown in Fig. 2, which may be interconnected to form a single fillable cavity.

As illustrated in Fig. 2, the inflatable item comprises several compartments 203A to 203E that have a relatively large cross-section and several compartments 203F to 203J that are relatively small in cross-section. Consequently, the relatively large compartments 203A to 203E are able to provide sufficient cushioning to the leg of the person while holding the persons leg and heel above a supporting surface 212, and the relatively small compartments 203F to 203J may be more easily folded around a person's leg 103 to provide a snug fit to their leg.

Furthermore, one or more of the smaller compartments, nearest to the edge 107 of the main body 102, such as compartment 203J may be folded such that it overlaps with the compartment 203A next to the opposite edge 109. In this way, the leg of a person may be completely surrounded by the inflated compartments of the inflatable item 201.

In the example of Fig. 2 the button 105 is provided on one of the compartments (203H) such that the compartment provides protection to a person's leg from any forces that the button may otherwise apply. In addition, the connecting portion 108 which defines the button hole 106 extends over inflated compartments 203I and 203J which protect the person's leg from any forces that the connecting portion would otherwise apply to the leg.

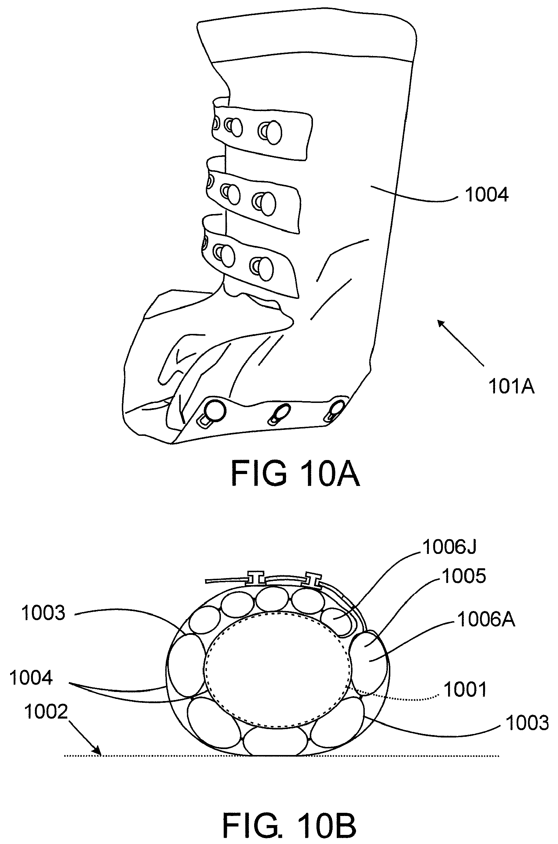

The inflatable item 201 may be used as shown in Fig.2, with possibly a sheet of material arranged between the inner surface of the inflatable item and the leg of the person. However, in alternative embodiments, (an example of which will be described below with respect to Figs. 10A and 10B) the apparatus further comprises an outer case in which the inflatable item is located. In this embodiment, the button is provided on the outer case, and the connection portion which defines the button hole, is formed as a part of the case. Thus, in this embodiment, the inflatable item may not have a button or an attachment portion attached directly to it.

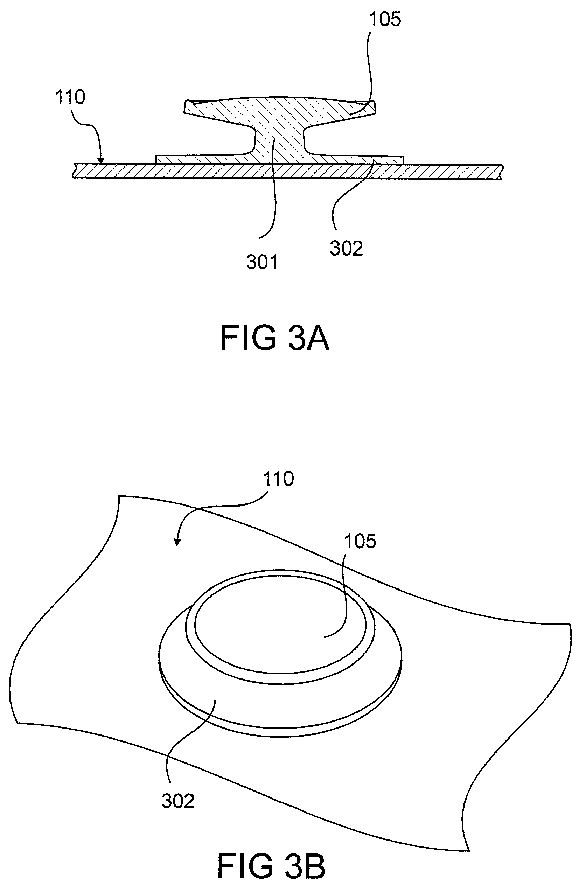

A more detailed view of the button 105 (separate from the button hole 106) is shown in the cross-sectional view of Fig. 3A and the perspective view of Fig. 3B. The button 105 is supported on a stalk 301 from a base 302. In this example, the button 105, the stalk 301 and the base 302 are formed in one piece. For example, the button 105, the stalk 301 and the base 302 may be formed in a moulding process from a polymeric material, such as polyvinyl chloride. Such buttons are known and available for the manufacture of garments such as raincoats. Advantageously, the button with integral stalk and base provides a continuous outer surface that is easily cleaned and which may be kept free of dirt and bacteria.

The base 302 is attached to the outer surface 110 of the main body. In the present example, the base 302 and the outer surface 110 are both formed of polymeric materials. In an example of a method of manufacturing the apparatus 101, the base 302 of the button is welded to the outer surface 110 of the main body 102, to attach the button to the main body 102, before the outer surface110 is joined to the inner surface 111 to form the main body.

In the present example, the button 105 has a circular shape, but it will be understood that buttons of various shape could be used instead. For example, the button 105 may be provided with an oblong shape, being longer in one direction that a perpendicular direction.

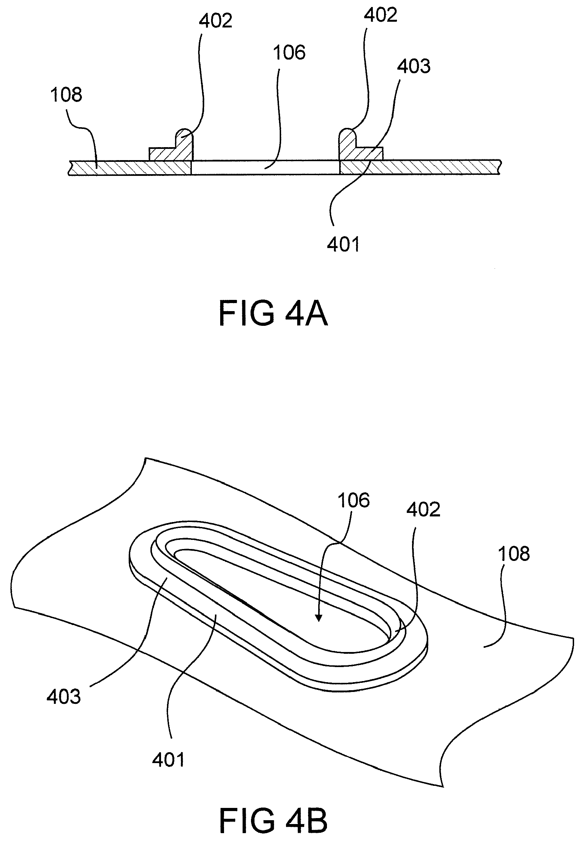

A more detailed view of the button hole 106 (separate from the button 105) is shown in the cross-sectional view of Fig. 4A and the perspective view of Fig. 4B. The button hole 106 is a hole formed in the connecting portion 108. However, as shown in Figs. 4A and 4B the connecting portion 108 may be reinforced around the button hole 106 by a reinforcing element 401 attached to the connecting portion. The reinforcing element 401 defines a hole through which the button 105 may be passed. The reinforcing element has a raised lip 402 along the edge of the hole and a base portion 403 surrounds the raised lip.

The reinforcing element 401 may be formed of a polymeric material, such as polyvinyl chloride, for example by moulding. Such reinforcing elements are known and commonly available for the manufacture of garments such as raincoats. The connecting portion 108 may be formed of a polymeric material, and the reinforcing element may be attached to the connecting portion by welding.

In the present example, the apparatus 101 comprises an inflatable item 201 formed of two similarly shaped sheets of polymeric material that are welded together along seams. The apparatus 101 is advantageously arranged to be reconfigurable between a flat configuration (which may be used for storage) and a three-dimensionally-shaped configuration (for use when supporting a person's leg).

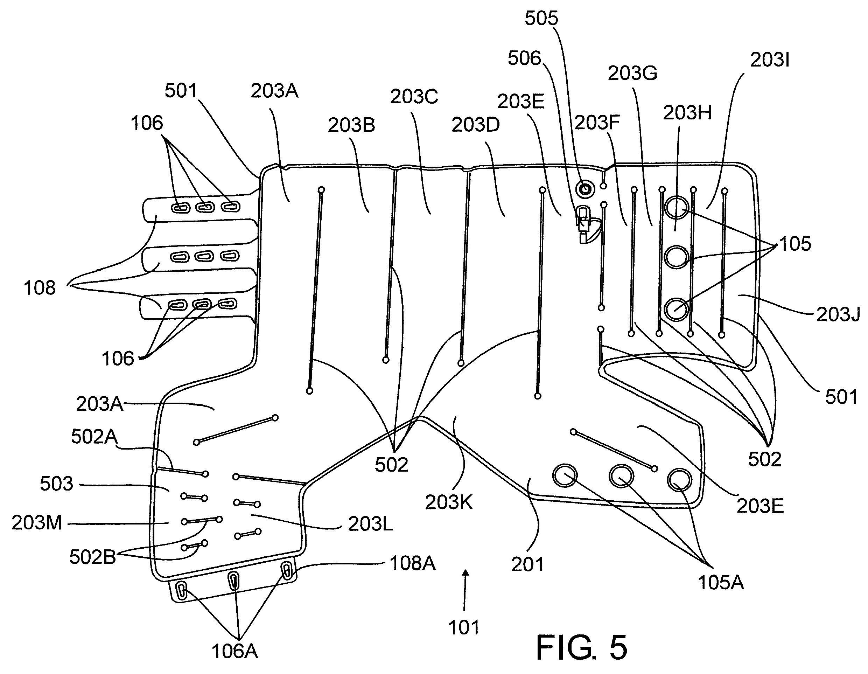

The apparatus 101 is shown in its flat configuration in the plan view of Fig. 5. The two sheets of material forming the inflatable item 201 are welded together along a seam 501 which extends completely around their edges to form a cavity that may be inflated with a gas. The two sheets are also welded along lines, such as lines 502, which divide the cavity up into a plurality of compartments, such as compartment 203A to 203J. The compartments 203B to 203C have one end open to a compartment 203K which, with lower portions of compartments 203E and 203A, is configured to extend around a person's foot during use.

A broken line 502A of welds separate the compartment 203K from a sole portion 503 of the inflatable item 201 which is intended to be located against a sole of a person's foot during use. The sole portion 503 comprises a plurality of welds 502B which divide up the sole portion 503 into a number of small compartments such as compartments 203L and 203M.

It will be understood that as well as defining the compartments of the inflatable item 201, the welds along the lines 502, 502A also define lines about which the inflatable item may be more easily folded when it is in its inflated state.

In the present example, the connecting portion 108 is one of three similar connecting portions 108, each in the form of a strap. In the present example the connecting portions 108 are formed of similar polymeric material to the two sheets forming the inflatable item 201. The connecting portions 108 may be permanently fixed to the inflatable item 201 during its manufacture by including one end of the connecting portions 108 in the weld which seals the two sheets together.

In the present example the apparatus 101 has three buttons 105 arranged in a line and fixed to the inflatable item 201 in compartment 203H. Each of the three buttons 105 is configured to be engaged with a button hole 106 on a respective one of the connecting portions 108. In the present example, the connecting portions 108 each comprise three button holes 106. In use any one of the three button holes 106 on a connecting portion may be used to fasten with a respective one of the buttons 105. By providing a plurality of button holes on the connecting portions, the apparatus 101 may be arranged to form a snug fit around calves of varying girth.

In the present example, the apparatus is provided with a second connecting portion 108A which extends along an edge of the sole portion 503 of the inflatable item 201. The connecting portion 108A is permanently attached to the sole portion 503 and this attachment may also be achieved by welding.

A fastening mechanism is provided to releasably attach the connecting portion 108A to a distant part of the compartment 203K. In the present example, the fastening mechanism comprises three buttons 105A and three button holes 106A. The button holes 106A are located on the connecting portion 108A and the buttons 105A are fixed to the compartment 203K of the inflatable item 201. The buttons 105A may be formed and fixed to the inflatable item 201 in the same manner as buttons 105. The button holes 106A may be formed in a similar manner to button holes 106.

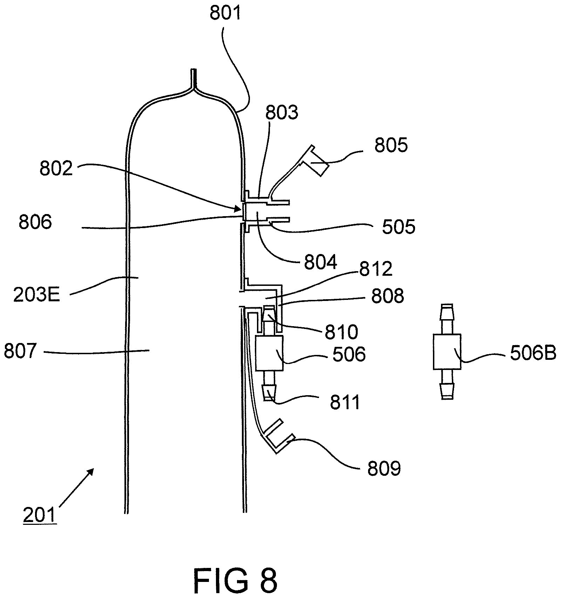

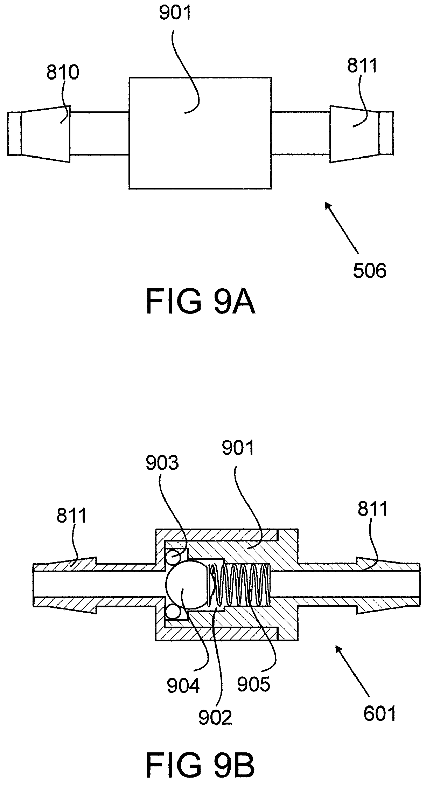

The inflatable item 201 may be provided with an inlet valve 505 to enable the inflatable item to be inflated. In the present example, the inflatable item 201 is also provided with a pressure release valve 506, but in alternative embodiments, no such pressure release valve is provided. The valves 505 and 506 will be described in further detail below with regard to Figs. 8 and 9.

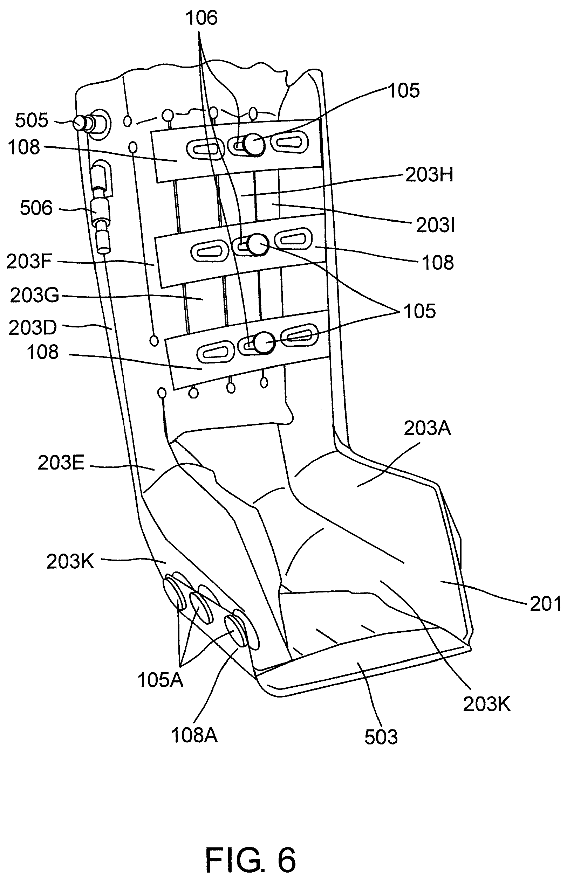

The apparatus 101 comprising inflatable item 201 is shown in a second configuration in the perspective view of Fig. 6. The inflatable item 201 is shown in Fig. 6 in its inflated state with the buttons 105A fastened with the button holes 106A of the connecting portion 108A. Therefore the sole portion 503 extends from one end of the compartment 203K to the other end of the compartment 203K.

To reconfigure the apparatus 101 from the flat configuration of Fig. 5 to the three-dimensionally-shaped configuration of Fig. 6, the inflatable item is inflated by filling with a gas via the inlet valve 505. The apparatus is folded about lines 502 and 502A and the buttons 105A are fastened with the button holes 106A of connecting portion 108A. Each of the buttons 105 located on the compartment 203H are fastened with one of the button holes 106 of the attachment portions 108.

The buttons 105 are also shown in Fig. 6 fastened with selected ones of the button holes 106 of the connecting portions 108. However, it will be understood that these buttons 105 are generally not fastened during use until the apparatus 101 has been located about a person's lower leg. That is, after inflating the apparatus 101 and fastening buttons 105A, the apparatus 101 is fitted to a person's leg by locating their calf within a channel formed by compartments 203A to 203E and locating the compartments 203A, 203E, 203K and the compartments of the sole portion 503 around the foot of the person. The compartments 203F to 203J may then be located over the shin of the person before the connecting portions 108 are fastened to buttons 105.

In an alternative example of an apparatus 101, the sole portion 503 may be permanently attached to each end of the compartment 203K, for example by replacing the buttons 105A and connection portion 108A with a permanent fixing means such as welding or adhesive. However, the illustrated embodiment has the advantage of allowing the apparatus to be stored in the flat configuration as shown in Fig. 5.

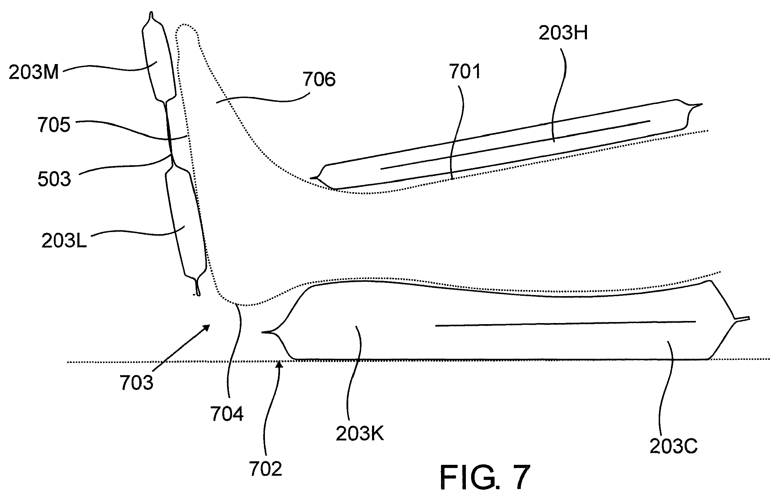

The apparatus 101 comprising inflatable item 201 is shown schematically in cross section in Fig. 7 supporting a lower leg 701 of a person above a surface 702. Typically the surface 702 would be an upper surface of a bed, such as a hospital bed.

As illustrated by Fig. 7, the compartment 203K, and the compartment 203C (along with the neighboring compartments 203B and 203D not shown in Fig. 7) provide support to the back of the calf of the person, such that the lower leg 701 is held above the surface 702.

The apparatus 101 defines a hole 703 which is bounded by the compartment 203K and the sole portion 503. The hole 703 is arranged to be positioned at the heel 704 of the person, so that neither the apparatus 101 nor the surface 702 apply any pressure to the heel.

The sole portion 503 is located against the sole 705 of the foot 706 of the person. Advantageously, the sole portion 503 comprises inflated compartments, such as compartments 203L and 203M, and therefore provides a cushioned surface to support the sole 705.

An end portion of compartment 203E of inflatable item 201, the inlet valve 505 and pressure release valve 506 are shown in cross-section in Fig. 8. The inlet valve comprises a moulded plastic inlet valve 505 of a type known in the art and commonly used on inflatable items. The inlet valve 505 may be formed of a polymer, such as polyvinyl chloride. The inlet valve 505 is welded to a sheet 801 which forms the inflatable item, at the position of a hole 802 in the sheet. The inlet valve 505 may comprise a tube 803 defining a passageway 804 and a stopper 805 for sealing off the passageway. The passageway may be provided with a diaphragm 806 configured to allow a gas to pass into the cavity 807 of the inflatable item 201 but prevent a gas from escaping from the cavity. The valve 505 may be configured to prevent gas escaping from the cavity unless the valve is deformed under an applied force, which may be applied, for example, by squeezing between fingers.

The pressure release valve 506 is attached to the sheet 801 forming the inflatable item 201 by a connection device 808 defining a passageway 812. The connection device 808 may be formed of a polymer suitable for welding to the polymer material of the sheet 801.

The pressure release valve 506 is configured to allow a flow of gas in a first direction when a pressure across the valve is above a threshold value and to prevent a flow of gas in the first direction when the pressure is below the threshold value.

The pressure release valve 506 has an inlet portion 810 configured to be connected to the passageway 812 of the connection device 808. With the inlet portion 810 fitted to the connection device, as shown in Fig. 8, the pressure release valve 506 is configured to allow gas to escape from the cavity 807 when pressure of gas within the cavity is more than the threshold value above the ambient air pressure and to prevent gas escaping from the cavity when pressure of gas within the cavity not more than the threshold value above ambient air pressure.

The inlet portion of the pressure release valve 506 is configured to be connected to the connection device 808 of the inflatable item 201 such that it is releasable. Consequently, the pressure release valve 506 may be removed and replaced with another valve such as pressure release valve 506B. The pressure release valve 506B may be formed in a similar manner to pressure release valve 506 but configured to allow a flow of gas when a pressure across the pressure release valve 506B is above a second threshold value that is different to the threshold value of the pressure release valve 506. Thus, by having several different valves, such as pressure release valve 506 and pressure release valve 506B, the pressure within the cavity may be maintained at or below a chosen threshold value above ambient air pressure.

In use, with the pressure release valve 506 fitted, the inflatable item 201 may be inflated by pumping a gas through the inlet valve 505. When pressure within the cavity exceeds the threshold value for the pressure release valve 506, the gas is able to escape through the pressure release valve 506. After pumping is stopped, the pressure within the cavity may drop until the pressure in the cavity 807 is equal to ambient atmospheric air pressure plus the threshold value for the pressure release valve 506.

With the stopper 809 left off the outlet, the apparatus 101 may be used to support a leg of a person. If the weight of the leg is applied to the inflatable item, the pressure of the gas within the cavity will rise, causing the pressure release valve 506 to open and release gas from the cavity 807 into the atmosphere until the pressure within the cavity is once again at the threshold value above ambient pressure. With the pressure in the cavity at the threshold value, the maximum pressure that the inflatable item applies to the person is then equal to the pressure in the cavity and therefore equal to the threshold value. Therefore, the pressure applied to the person is set by the threshold value of the valve.

In examples, the valves 506 and 506B are configured to open when the pressure difference between the inlet and the outlet is greater than a pressure difference of between 0.1 pounds per square inch (6.9 mbar) and 1 pound per square inch (69 mbar). Therefore, the threshold value is such that the pressure in the cavity is greater than ambient atmospheric pressure by between 0.1 pounds per square inch (6.9 mbar) and 1 pound per square inch (69 mbar).

In some of these examples, the valves 506 and 506B are configured to open when the pressure difference between the inlet and the outlet is greater than a pressure difference of between 0.15 pounds per square inch (10.3 mbar) and 0.8 pounds per square inch (55 mbar). Therefore, the threshold value is such that the pressure in the cavity is greater than atmospheric pressure by between 0.15 pounds per square inch (10.3 mbar) and 0.8 pounds per square inch (55 mbar).

In some of these examples, the valves 506 and 506B are configured to open when the pressure difference between the inlet and the outlet is greater than a pressure difference of between 0.2 pounds per square inch (14 mbar) and 0.5 pounds per square inch (35 mbar).

An example of a pressure release valve 506 for use with the apparatus 100 is a check valve. An example of a suitable valve is shown in the front view and cross-sectional view of Figs. 9A and 9B.

The pressure release valve 506 comprises a main body 901 between the inlet portion 810 and the outlet portion 811. The inlet portion and outlet portion define passageways that extend inwards to a central chamber 902 in the main body 901. The central chamber contains an 0-ring which forms a valve seat 903 and a ball 904 configured to rest against the valve seat 903 to form a seal. The central chamber 902 also contains a compression spring 905 configured to urge the ball against the valve seat 903 to maintain the valve in the closed configuration shown in Fig. 9B. However, when sufficient gas pressure is applied to the inlet side of the ball 904, the ball is forced against the force of the spring 905 away from the valve seat to allow gas to pass around the ball and through the valve.

As will be understood from Fig. 9B, the pressure release valve 506 is also configured to prevent any flow of gas from its outlet to its inlet, and therefore the inflatable item cannot be inflated through the pressure release valve 506.

An alternative example of an apparatus 101 A for supporting a leg of a person laid on a surface 1002 is shown in the perspective view of Fig 10A and the cross-sectional view of Fig. 10B. The apparatus 101A comprises an inflatable item 1003 which may have a form identical to the inflatable item 201 of apparatus 101. The inflatable item 1003 may therefore have a cavity 1005 which is divided into a plurality of compartments, such as compartments 1006A, 1006C and 1006J, in the same manner as inflatable item 201.

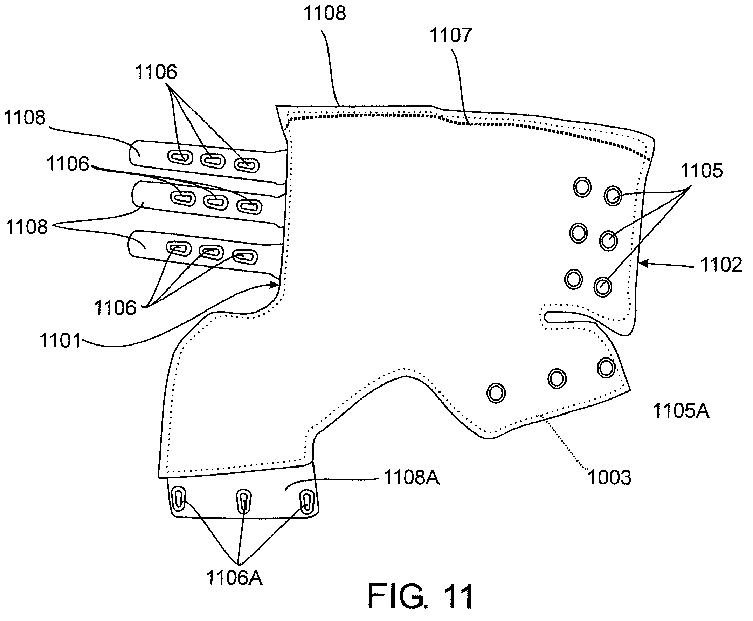

Unlike the apparatus 101, the apparatus 101A also comprises a case 1004 which covers the inflatable item 1003. The case 1004 is shown in an unfastened, flat configuration in Fig. 11. The case 1004 is in the form of a contoured bag having a sealing mechanism, such as a zip (or zipper) 1007 extending across an upper edge 1008. In its open configuration, the zip 1007 allows the inflatable item to be inserted within the case 1004. The inflatable item 1003 may be inflated, for example by a suitable pump, either before its insertion or afterwards. The zip may then be closed to keep the inflatable item in position within the case.

The case 1004 may be formed of one or more flexible sheets of a continuous (that is, not woven or knitted) polymer material that may be seam welded to form the bag shape. The outline of the case 1004 (as shown in Fig.11) is contoured to provide a close fit around the inflatable item 1003.

The case 1004 is provided with connecting portions 1108, similar to the connecting portions 108 of apparatus 101, attached to one edge 1101 of the case. The connecting portions 1108 each comprise several button holes 1106. The case also has several buttons 1105 arranged in rows near to another edge 1102 of the case. The number of rows is arranged to be equal to the number of connecting portions 1108 (which in the present example is three).

The case 1004 also comprises a further connecting portion 1108A having several button holes 1106A which are configured to engage buttons 1105A.

The buttons 1105 and 1105A may be of the same moulded construction as the buttons 105 of apparatus 101. The button holes 1106 and 1106A may have reinforcing element like those of button holes 106.

By fastening buttons 1105A and button holes 1106A and by fastening buttons 1105 with button holes 1106 the apparatus 101A may be fitted to a leg of a person in a similar manner to the apparatus 101. Thus, the apparatus 101A may perform the same function as apparatus 101.

It should also be noted that the case 1004 is formed of a water impermeable sheet material and the buttons 1105 and 1105A are formed of a polymer material that is also water impermeable. Consequently the case 1004 provides an outer surface (including around the buttons) that may be cleaned and dried by wiping.

The inflatable item 1003 may be identical to inflatable item 201, but because the inflatable item 1003 is located within the case 1004, the inflatable item may be made without buttons (105, 105A) or connecting portions (108, 108A).

Although embodiments of the present invention have been described in the preceding paragraphs with reference to various examples, it should be appreciated that modifications to the examples given can be made without departing from the scope of the invention as claimed. For example, although the above described inflatable members have been provided with inlet valves, alternative examples may comprise inflatable members that are inflated and sealed during manufacture, such that further inflation or deflation is not possible. Also, the main described embodiments each have an inflatable member, but other means for providing the required deformability and resilience may be used. For example, the interior of the apparatus may be provided with a resilient, deformable foam material in place of an inflatable item.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

Whilst endeavoring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. An apparatus (101, 101 A) for supporting a leg of a person laid on a surface, the apparatus comprising:
a main body (102) for location around a leg of a person; and
a fastening mechanism (104) configured to connect different parts of the main body for attaching the apparatus to a leg,
wherein the fastening mechanism comprises an engaging element (105) and a hole (106) configured to receive the engaging element.

2. An apparatus according to claim 1, wherein the engaging element (105) is a button and the hole (106) is a button hole.

3. An apparatus according to claim 2, wherein the button (105) is formed of a polymeric material.

4. An apparatus according to claim 2 or claim 3, wherein the button (105) is supported on a stalk (301) from a base (302); and the button, the stalk and the base are formed in one piece.

5. An apparatus according to any one of claims 2 to 4, wherein the fastening mechanism (104) comprises a connection portion (108) formed of a polymeric material attached to the main body (102), and the button hole (106) is formed in the connection portion.

6. An apparatus according to claim 5, wherein a reinforcing element (401) is attached to the connection portion (108) at the location of the button (105) hole (106).

7. An apparatus according to any one of claims 1 to 6, wherein the main body (102) comprises a left portion and a right portion that are configurable to extend on either side of a space for receiving a foot, and the main body (102) further comprises a sole portion (503) configurable to extend between the left portion and the right portion for supporting a sole of a foot.

8. An apparatus according to claim 7, wherein the main body (102) comprises an inflatable item (201) and the sole portion (503) is inflatable.

9. An apparatus according to claim 7 or claim 8, wherein the sole portion (503) is releasably attachable to the left portion and/or the right portion by a second fastening mechanism (105A and 106A).

10. An apparatus according to claim 9, wherein the second fastening mechanism (105A and 106A) comprises buttons (105) and button holes (106).

11. An apparatus according to claim 9 or claim 10, wherein the main body (102) is reconfigurable between a first configuration in which the second fastening mechanism (105A and 106A) is undone and the main body (102) is able to lay flat and a second configuration for use.

12. An apparatus according to any one of claims 1 to 11, wherein the main body (102) comprises an inflatable item (201) or the main body (102) comprises a case (1004) containing an inflatable item (201).

13. An apparatus according to claim 12, wherein the inflatable item (201) has an outer wall defining a cavity (202) for receiving a gas, and the inflatable item is provided with a pressure release valve (506) configured to allow gas to escape from the cavity (202) when pressure of gas within the cavity is more than a threshold value above the ambient air pressure and to prevent gas escaping from the cavity when pressure of gas within the cavity not more than the threshold value above ambient air pressure.

14. A method of manufacturing the apparatus of any one of claims 1 to 13, wherein the engaging element (105) is formed of a first polymer material and the main body (102) has an outer surface (110) comprising a second polymer material and the method comprises welding the engaging element to the outer surface.

15. The method of claim 21, wherein the engaging element (105) comprises a button (105) supported on a stalk (301) from a base (302) and the welding comprises welding the base (302) to the outer surface (110).
